# DEMANDE DE BREVET EUROPEEN

(11) **EP 1 391 184 A1**
(43) Date de publication de la demande: **25.02.2004**
(21) Numéro de dépôt: 02405698.8
(22) Date de dépôt: 16.08.2002
(51) Int. Cl.: A61F 2/06

(54) **Structure tubulaire multicouches extensible et procédé de fabrication**

(71) Demandeur: Endosense Sàrl, 1242 Satigny (CH)
(72) Inventeur: Vitali, Verin, E., 1225 Chêne-Bourg (CH); Papirov, Igor, I., Kharkov-108 (UA)
(74) Mandataire: Kiliaridis, Constantin

(57) **Abrégé**

L'invention concerne une structure tubulaire multicouches extensible radialement destinée à être utilisée comme stent et le procédé de fabrication. La structure comprend une couche extérieure (1) et une couche intérieure (2) solidement accrochées l'une à l'autre. L'une des couches (2) est munie dans son épaisseur des creusures (3)et l'autre des perforations (4). Les creusures (3) permettent, d'une part, d'influencer les propriétés mécaniques du stent et, d'autre part, de loger un médicament pour le traitement local du vaisseau dans lequel le stent sera disposé.

## Description

La présente invention concerne une structure tubulaire multicouches extensible destinée à être utilisée comme stent comprenant au moins deux couches solidement accrochées l'une à l'autre, ainsi qu'un procédé pour sa fabrication.

I1 est connu depuis plusieurs années d'utiliser des dispositifs implantables pour le traitement de certaines maladies du corps humain ou animal. Ainsi on introduit partiellement ou complètement un tel dispositif dans l'oesophage, la trachée, le côlon, le système vasculaire, le conduit urinaire, etc. Dans le système vasculaire, lorsqu'on veut introduire un tel dispositif appelé communément stent, nous avons besoin d'un cathéter, d'un ballon et d'un guide filiforme ou similaire. Le dispositif lorsqu'il est positionné à l'endroit que l'on doit traiter s'étend radialement pour maintenir les parois du vaisseau. En fonction du traitement souhaité, le stent doit rester en place ou être retiré après la période de traitement. Certains des stents qui restent en place sont munis d'un médicament qui doit être diffusé pendant une certaine période afin, d'une part, traiter la lésion et, d'autre part, pour remédier aux lésions qui peuvent subvenir par le fait que le vaisseau, lors de l'introduction du stent a été perturbé. Le stent, notamment celui qui doit rester à l'intérieur du tube doit présenter certaines caractéristiques mécaniques quasi contradictoires, à savoir : être petit, léger et surtout, après avoir été détendu radialement, supporter la pression des parois du vaisseau ou du tube à l'intérieur duquel il se trouve sans s'affaisser. Il faut donc, d'une part, qu'il soit suffisamment rigide et résistant radialement pour éviter un affaissement intempestif et, d'autre part, il faut qu'il puisse être extensible afin qu'il occupe la place qui est nécessaire pour le traitement à l'intérieur du vaisseau ou du tube humain ou animal.

La présente invention a pour but de proposer un tel dispositif lequel, d'une part présente de bonnes caractéristiques mécaniques allant dans le sens précité et, d'autre part, si on le désire, il peut contenir un médicament pour une diffusion à l'endroit du traitement.

La structure tubulaire multicouches selon l'invention est définie par la partie caractérisante de la revendication 1.

L'intérêt de cette structure multicouches est le fait qu'elle est constituée d'au moins deux couches différentes qui peuvent éventuellement présenter des caractéristiques mécaniques différentes par leur nature, mais en tout cas par leur structure, à savoir le fait que l'une des couches, préférablement la couche située à l'intérieur, est munie de creusures donnant des caractéristiques mécaniques différentes de l'autre couche.

Selon les variantes d'exécution, l'une des couches, préférablement la couche extérieure, peut également être munie de perforations radiales dont le but et surtout lorsque le stent comprend un médicament de permettre sa diffusion vers l'extérieur.

Selon une autre variante d'exécution, les creusures sont orientées soit vers l'intérieur de la couche située à l'intérieur, soit vers l'extérieur en fonction de l'utilisation souhaitée.

Selon une autre variante d'exécution la structure comprend au moins trois couches dont préférablement au moins une est munie de perforations radiales

L'invention concerne également le procédé de fabrication d'un tel stent tel que défini principalement par les revendications de procédé, et notamment les revendications 9 et 12.

La différence essentielle entre les deux revendications 9 et 12 est le fait que dans le cas de la revendication 9 , la structure est fabriquée en partant de deux feuilles métalliques pour former le tube qui aboutira au stent, tandis que dans l'autre cas, on part de deux tubes métalliques dont le diamètre extérieur de l'un est légèrement inférieur au diamètre intérieur de l'autre afin que l'on puisse glisser l'un des tubes dans l'autre. Les différentes étapes de formation sont quasiment identiques comme il sera décrit par la suite à l'aide du dessin annexé.

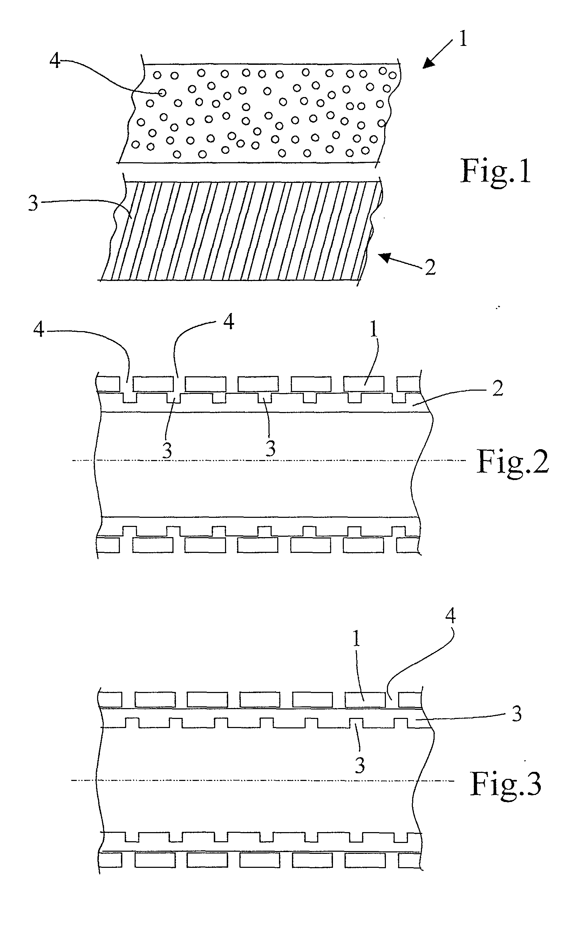

La figure 1 représente deux feuilles métalliques en perspective.

Aux figures 2 et 3, nous avons représentés deux variantes d'un tube formé à partir de deux feuilles de la figure 1, mais avant l'usinage pour obtenir le maillage du type stent.

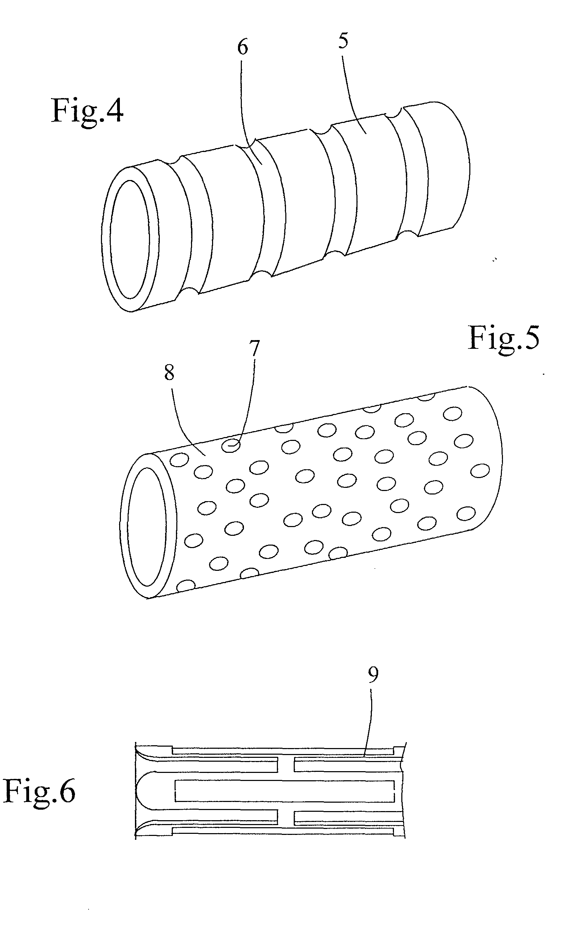

A la figure 4 nous avons représenté, en perspective, une variante du tube intérieur selon l'autre variante du procédé et à la figure 5 le tube extérieur et,

A la figure 6 nous avons présenté vue de côté, une représentation connue d'un maillage du type stent.

A la figure 1, nous avons deux feuilles métalliques 1 et 2 qui peuvent être choisies dans des matériaux biocompatibles du type Ta ou acier 316L ou de l'Elgiloy 40%, Pt-Ir alliage etc. l'épaisseur de ces feuilles est de l'ordre de 50 microns. Dans la feuille numéro 2 qui constituera la couche intérieure du stent, on grave sur sa surface des creusures. Ces creusures peuvent être sous la forme de lignes droites ou toutes autres configurations. On peut prévoir que ces lignes forment des dessins périodiques dont la période est de l'ordre de 50 à 60 microns. Leur profondeur peut être de l'ordre de 40% de l'épaisseur de la feuille soit environ 20 microns.

La feuille numéro 1 qui constituera la couche extérieure est, dans le cas présent, munie de perforations 4 mais on peut aussi la laisser telle quelle. L'épaisseur de la feuille 1 est également de l'ordre de 50 microns. Les feuilles 1 et 2 peuvent être faites de deux matériaux différents en fonction des résultats mécaniques que l'on désire obtenir. Par la suite, les deux surfaces des deux feuilles qui vont se mettre l'une contre l'autre sont traitées pour permettre un accrochage de deux feuilles de sorte à constituer pratiquement une pièce unique et qu'elles ne puissent pas se séparer lors des étapes de fabrication suivantes. On peut traiter les deux surfaces par sablage ou au plasma ou toutes autre méthode similaire permettant lorsqu'on mettra les feuilles l'une contre l'autre avec les deux surfaces se faisant face, d'obtenir un accrochage intime.

Selon une variante d'exécution préférée, on peut déposer sur l'une des surfaces traitées une couche métallique d'une épaisseur maximale de 1 micron afin de améliorer l'accrochage entre les deux surfaces.

Par la suite, on superpose les deux feuilles avec les deux faces l'une contre l'autre et on effectue un laminage à chaud et sous-vide de la structure ainsi obtenue. Par la suite on forme par usinage, par exemple au moyen d'un laser ou autre procédé similaire, un maillage propre au stent, c'est-à-dire on forme des trous traversant les deux couches. Par la suite, on plie la structure ainsi obtenue pour former un tube lequel est soudé, par exemple par laser, le long de la génératrice, et par la suite découpée à la longueur voulue pour former les stents. Le stent obtenu, par exemple, peut être celui représenté à la figure 6 qui n'est qu'une variante de maillage des stents connus.

Si on le désire, on peut remplir les creux qui subsistent correspondant aux creusures dans l'épaisseur de la couche inférieure, voire même les perforations avec un médicament ou association des médicaments qui sera diffusé par la suite à l'endroit du traitement.

Il y a deux manières de former le stent dans le cas présent et notamment lors de l'assemblage de deux feuilles l'une contre l'autre on peut les assembler soit comme représenté à la figure 2 en traitant la surface de la feuille 2 munie avec les creusures 3 et les assembler à la surface traitée de la feuille supérieure 1, soit, comme à la figure 3, les creusures sont dirigées vers l'intérieur du stent.

Le stent ainsi obtenu présente de bonnes caractéristiques de résistance radiale à l'affaissement et également des possibilités d'expansion radiales, qualités qui sont nécessaires pour un stent. En effet lors du laminage la limite d'élasticité de chaque couche augmente en fonction de la diminution de l'épaisseur de chaque couche ce qui améliore les qualités précités du stent. Il s'est avéré qu'en utilisant un laminage d'une structure du type "sandwich" d'une épaisseur donnée on obtient une limite d'élasticité supérieure à la limite d'élasticité obtenue après le laminage d'une couche unique ayant la même épaisseur que la structure "sandwich". Pour cette raison on peut appliquer par analogie le même procédé de fabrication en utilisant au moins trois couches dont de préférence au moins une est munie de perforations radiales et les autres de creusures.

A la figure 4, nous avons représenté deux tubes d'une épaisseur également de l'ordre de 50 microns. Le tube 5 qui sera le tube intérieur présente un diamètre extérieur légèrement inférieur au diamètre intérieur du tube 6, de sorte qu'on puisse glisser le tube 5 dans le tube 6. Comme précédemment, la surface extérieure du tube 5 est munie de creusures 6 qui peuvent avoir des formes différentes, par exemple des anneaux, des cercles ou des ellipses ou toutes autres figures similaires, la profondeur des creusures étant toujours déterminée en fonction des propriétés mécaniques désirées. La profondeur peut être de l'ordre de 40% de l'épaisseur de la paroi du tube soit environ 20 microns. Ensuite on traite cette surface par sablage ou au plasma ou toutes autre méthode similaire afin qu'on puisse par la suite obtenir un accrochage étroit entre les deux tubes.

Le tube 6 peut être muni de perforations radiales 7. Après avoir traité la surface extérieure du tube 5, on glisse le tube 5 dans le tube 6 et on applique un étirage à chaud et sous-vide des deux tubes de sorte à obtenir un accrochage mécanique des deux tubes formant un seul élément qui ne risque pas de se défaire par la suite. Il est également possible, comme dans le cas précédent, de déposer sur la surface extérieure du tube 5 une couche d'un métal d'une épaisseur maximum de 1 micron afin de faciliter cet accrochage entre les deux surfaces, à savoir la surface intérieure du tube 6 et la surface extérieure du tube 5.

Par la suite, le tube obtenu après l'étirage est usiné par des moyens connus tel que laser, attaque chimique etc. pour obtenir un maillage du type stent, comme par exemple celui qui est représenté à la figure 6. Il suffit par la suite de découper le tube ainsi obtenu à la longueur voulue pour obtenir les stents. Les qualités de ce stent sont les mêmes que celles obtenues précédemment par un moyen tout à fait similaire, la seule différence étant le fait que nous partons de deux feuilles métalliques plutôt que de deux tubes. Dans le cas présent, il est également possible de remplir les creusures 6 et les éventuelles perforations 7 avec un médicament pour qu'il soit diffusé par la suite lorsque le stent sera disposé dans le corps. Comme mentionné précédemment, les matériaux utilisés pour les deux tubes peuvent être choisis parmi ceux mentionnés précédemment, les deux tubes pouvant être du même matériau ou de deux matériaux différents afin de tirer profit de leurs caractéristiques mécaniques liées à leur nature et forme particulière.

La remarque précédente concernant l'augmentation de la limite d'élasticité s'applique aussi à cette exécution et on pourrait prévoir de structures à au moins trois couches en appliquant par analogie le même procédé

## Revendications

1. Structure tubulaire multicouches extensible radialement destinée à être utilisée comme stent comprenant au moins deux couches, **caractérisée par le fait qu'**au moins une des couches est munie dans son épaisseur de creusures.

2. Structure selon la revendication 1, **caractérisée par le fait qu'**au moins une des couches est munie de perforations radiales.

3. Structure selon l'une des revendications 1 ou 2, **caractérisée par le fait que** lesdites creusures sont situées sur la surface intérieure de ladite couche.

4. Structure selon l'une des revendications 1 ou 2, **caractérisée par le fait que** lesdites creusures sont situées sur la surface latérale extérieure de la couche située à l'intérieur de la structure.

5. Structure selon l'une des revendications 1 à 4, **caractérisée par le fait que** lesdites creusures sont remplies avec un médicament.

6. Structure selon l'une des revendications 1 à 5, **caractérisée par le fait que** le matériau utilisé pour lesdites couches est du Ta ou de l'acier 316L ou de l'Elgiloy (40%), ou du Pt-Ir alliage ou tout autre métal ou alliage biocompatible.

7. Structure selon la revendication 1, **caractérisée par le fait qu'**il comprend au moins trois couches.

8. Structure selon la revendication 7, **caractérisée par le fait qu'**au moins une couche est munie de perforations radiales.

9. Procédé pour la fabrication d'une structure selon la revendication 1, **caractérisé par le fait que** les deux couches sont formées à partir de deux feuilles métalliques selon les étapes suivantes :
a) formation de creusures sur la surface d'une feuille ;
b) traitement d'une des surfaces de chaque feuille par sablage ou au plasma;
c) superposition de deux feuilles avec leurs faces traitées l'une contre l'autre et laminage à chaud sous-vide ;
d) usinage de l'ensemble de deux feuilles accrochées pour obtenir un maillage propre aux stents ;
e) formation d'un tube par enroulement et soudure le long de la génératrice formant le joint,
f) découpe du tube selon la longueur souhaitée pour obtenir des stents .

10. Procédé selon la revendication 9, **caractérisé par le fait que** l'autre feuille est munie de perforations avant le traitement d'une de ses surfaces.

11. Procédé de fabrication de la structure selon la revendication 1, les deux couches étant constituées par un premier tube métallique et un second tube métallique, le diamètre extérieur du second tube métallique étant inférieur au diamètre inférieur du premier pour qu'ils puissent être glissés l'un dans l'autre, **caractérisée par** les étapes suivantes :
a) on forme dans la paroi extérieure du second tube des creusures ;
b) on traite la surface extérieure du second tube par sablage ou au plasma;
c) étirage sous-vide et à chaud du tube obtenu après avoir glissé le second tube dans le premier ;
d) usinage du tube unique ainsi obtenu pour former une structure présentant un maillage propre aux stents ;
e) découpe du tube ainsi obtenu à la longueur souhaitée pour obtenir des stents.

12. Procédé selon la revendication 11, **caractérisé par le fait que** le premier tube est muni de perforations avant son assemblage au second.

13. Procédé selon l'une des revendications 9 ou 11, **caractérisé par le fait qu'**après avoir traité la ou les surfaces qui entreront en contact pour assurer leur accrochage, on dépose sur l'une desdites surfaces une fine couche d'une épaisseur maximale de 1 micron d'un métal permettant d'améliorer l'accrochage étroit de deux surfaces pour éviter la séparation de deux tubes..

14. Procédé selon l'une des revendications 9 à 13, **caractérisé par le fait que** l'on remplit les creusures avec un médicament.

15. Procédé selon l'une des revendications 9 ou 11, **caractérisé par le fait que** les creusures forment des figures périodiques dont la période est de 50 à 60 microns.

16. Procédé selon l'une des revendications 10 ou 12, **caractérisé par le fait que** lesdites perforations forment des figures périodiques dont la période est de 50 ou 60 microns.
